# EUROPEAN PATENT APPLICATION

(11) **EP 4 162 905 A1**
(43) Date of publication of application: **12.04.2023**
(21) Application number: 21817613.9
(22) Date of filing: 02.06.2021
(51) Int. Cl.: A61F 9/007

(54) **SURGICAL INSTRUMENT FOR VITREORETINAL SURGERY**

(30) Priority: 04.06.2020 ES 202030526
(71) Applicant: Marticorena Salinero, José Joaquín, 15706 Santiago de Compostela (ES)
(72) Inventor: Marticorena Salinero, José Joaquín, 15706 Santiago de Compostela (ES)
(74) Representative: López Camba, Emilia
(86) International application number: PCT/ES2021/070393
(87) International publication number: WO 2021/245309

(57) **Abstract**

Instrument for vitreoretinal surgery comprising a handle (1) of thermoplastic polymer; a stainless steel cannula (3) arranged at the distal end of the handle (1); a stainless steel stem (4) running through the interior of the cannula (3); three adjoining petal-shaped nitinol handles (5) attached at the distal end of the stainless steel stem (4) running through the interior of the cannula; and a movable button (2) embedded in the handle (1) of the instrument and sliding on a rail so that by acting on the movable button they allow the surgeon to extend and retract the wire handles. It has a design that allows the design of which that allows the extraction of intraocular foreign bodies of different shape, size and composition as well as manipulating the dislocated lens to the vitreous cavity.

## Description

### Object of the invention

The object of the present invention, as the title of the invention establishes, is a surgical instrument for ophthalmological use in surgery of the posterior segment of the eyeball, also known as vitreoretinal surgery, capable of being introduced through small-caliber valved trocars.

Vitreoretinal surgery allows the treatment of complications of surgery of the anterior segment of the eye, such as dislocation of the lens into the vitreous cavity during cataract surgery, or the treatment of eye trauma with the presence of an intraocular foreign body.

The present invention is characterised by the special design, configuration and nature of the materials of each and every one of the elements that form part of this surgical instrument and make it possible to extract intraocular foreign bodies of different morphology, size and composition, as well as to manipulate and stabilise the entire nucleus of the lens or fragments thereof dislocated towards the vitreous cavity so that the surgeon can use the vitreotome or phaco-fragmenter more safely and efficiently.

In view of the foregoing, the present invention falls within the scope of surgical instruments for vitreoretinal surgery within the specialty of ophthalmology.

### Background

Within the medical-surgical specialty of ophthalmology, vitreoretinal surgery involves a series of surgical procedures that involve performing a vitrectomy via pars plana by introducing surgical instruments into the vitreous cavity through small-caliber valved trocars (0.573 mm, 0.455 mm, 0.360 mm) (23 gauge, 25 gauge or 27 gauge). Among the different indications for vitreoretinal surgery are trauma with the presence of an intraocular foreign body or the dislocation of the lens towards the vitreous cavity.

The extraction surgery of an intraocular foreign body is always a complex situation in the context of serious eye trauma. The size, shape and composition of the foreign body can be variable, which implies a series of additional limitations and difficulties due to the impossibility of extracting it with the available instruments, especially when the size of the foreign body exceeds the maximum opening of the branches of the clamp used. None of the clamps currently available has a caliber that allows it to be introduced through valved vitrectomy trocars. To this must be added that currently available intraocular foreign body extraction tweezers have a maximum branch opening of 2.00 mm. If it is a nonmagnetic foreign body it is also not possible to use a vitreoretinal magnet to manipulate it and facilitate its removal. It is particularly difficult to extract shot pellets, since they are spherical foreign bodies and cannot be caught with a conventional clamp. Nowadays there is a clamp that allows to extract pellets of maximum caliber 00 (4.50 mm in diameter) which implies a large scleral incision, without it being possible to introduce it by trocars. It is not possible to catch larger pellets.

On the other hand, the dislocation of the lens or fragments thereof towards the vitreous cavity may be of traumatic origin, or as a consequence of cataract surgery complications. When it is necessary to perform a vitrectomy to extract the dislocated nucleus towards the vitreous cavity, an important drawback is the lack of an instrument to stabilise the nucleus while using the vitreotome or the phaco-fragmenter. Furthermore, the continuous movement of the nucleus or its fragments poses a significant risk of damage to the retina. Currently, there is no surgical instrument that allows the manipulation or stabilization of the lens nucleus or its fragments during vitrectomy.

The objective of the present invention is to allow the manipulation and extraction of different intraocular foreign bodies, overcoming the limitations of the instruments currently available, as well as to overcome the difficulties involved in not having a surgical instrument that stabilizes the lens or its dislocated fragments towards the vitreous cavity during a vitrectomy, developing a surgical instrument such as the one described below and which is essentially contained in the first claim.

### Description of the invention

The object of the present invention is a surgical instrument for vitreoretinal surgery comprising:
- a thermoplastic polymer handle,
- a stainless steel cannula disposed at the distal end of the handle,
- a stainless steel stem that runs through the inside of the cannula,
- three curved smooth nitinol handles in the shape of a petal joined at the distal end of the stainless steel stem that runs through the inside of the cannula,
- a movable button, embedded in the handle of the instrument and sliding on a rail. This button is attached to the proximal end of the stainless steel stem.

Where all the above elements allow the surgeon to extend and retract the nitinol wire handles by means of the movable button in such a way that the basket formed by the handles is closed and inside the cannula by moving the movable boot towards the proximal area, while by moving the movable button towards the distal area it is possible to gradually extend and open the basket formed by the 3 nitinol handles to the outside of the cannula.

The flexibility and memory of the nitinol wire added to the mechanism that allows the handles to be introduced into the cannula or extended as a basket within the vitreous cavity makes it possible to use this surgical instrument through valved trocars of small caliber when the objective is to trap an intraocular foreign body of variable shape, size and composition

Thus, the instrument is also able to catch with its open handles the entire nucleus of the dislocated lens or its fragments, while at the same time stabilizing and manipulating it inside the cavity when retracting the handles. In this way, the surgeon can "shave" the nucleus of the lens with the vitreotome or phaco-fragmenter, using 23 gauge trocars, far from the retina, in a safer, faster and more efficient way.

In summary, the surgical instrument object of the application is multipurpose, the design of which allows the extraction of various intraocular foreign bodies of different shapes.

Unless indicated otherwise, all the technical and scientific elements used in this specification have the meaning usually understood by a person skilled in the art to which this invention belongs. In the practice of this invention, methods and materials similar or equivalent to those described in the specification may be used. In the description and claims, the word "comprises" and its variants do not intend to exclude other technical features, additives, components or steps. For persons skilled in the art, other objectives, advantages and features of the invention will be inferred partly from the description and partly from the practice of the invention.

### Explanation of the figures

In order to complement the description being made herein, and to aid a better understanding of the characteristics of the invention, in accordance with a preferred practical embodiment thereof, said description is accompanied, as an integral part thereof, by a set of drawings where, in an illustrative and non-limiting manner, the following has been represented:
- Figure 1. General representation of the instrument.

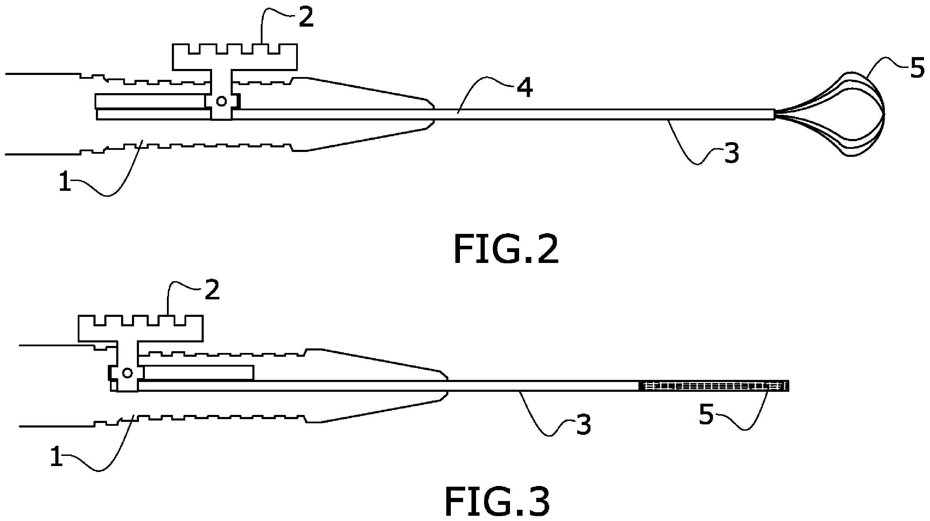

- Figure 2. Interior of the instrument with the three nitinol handles deployed.
- Figure 3. Instrument with the three nitinol handles collected inside the cannula.

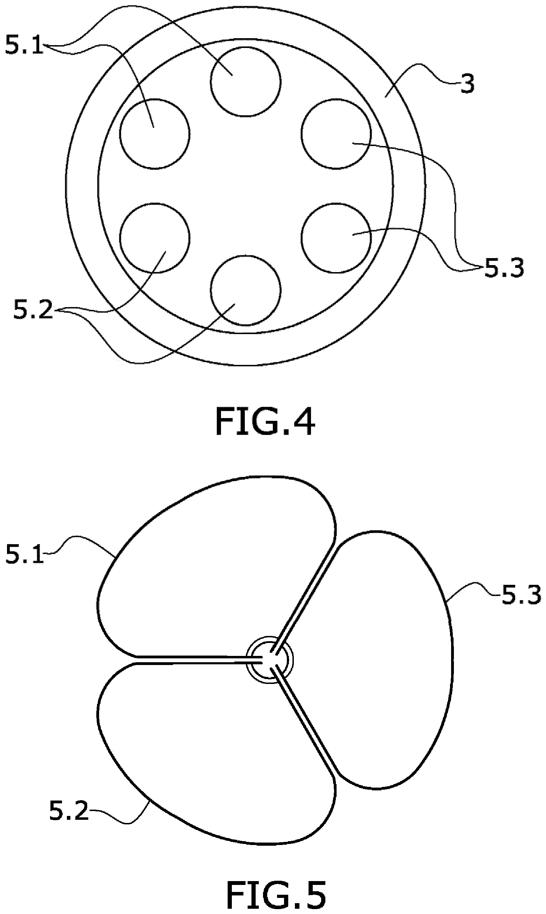

- Figure 4. Plan view of the end of the steel cannula and the arrangement of the nitinol handles.
- Figure 5. View of the curved petal-shaped nitinol handles and their relative position.

### Preferred embodiment of the invention

In view of the figures, a preferred embodiment of the proposed invention is described below.

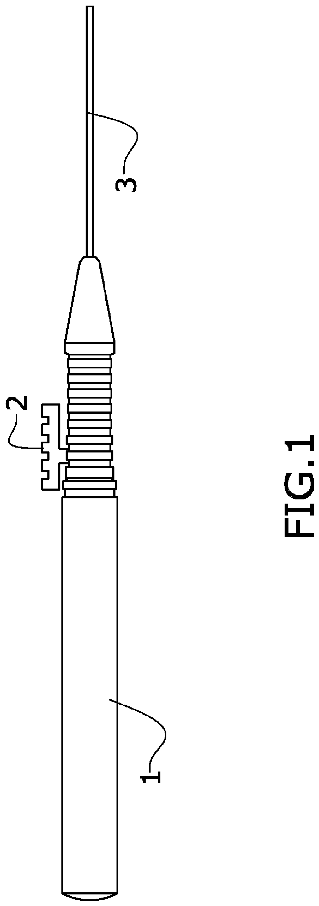

Figure 1 shows that the instrument object of the invention comprises a handle (1) of thermoplastic polymer; a stainless steel cannula (3) arranged at the distal end of the handle (1); a stainless steel stem (4) that runs through the inside of the cannula (3); three adjoining petal-shaped nitinol handles (5) joined at the distal end of the stainless steel stem (4) that runs through the inside of the cannula; a mobile button (2) embedded in the handle (1) of the instrument that slides on a rail. In one possible embodiment, the cannula (3) has an external caliber of 0.573 mm in diameter (23 gauge), an internal diameter of 0.47 mm and a length of 33 mm. The 3 handles (5) are made of round and smooth monofilament nitinol wire of 0.113 mm diameter (37 gauge). The stainless steel stem, in a possible embodiment, can have a diameter of 0.404 mm (26 gauge) and a length of 52.00 mm, at the distal end of which the nitinol handles are attached. The length of the rail on which the mobile button slides can have a length of 13.00 mm.

In a preferred but non-limiting embodiment, the maximum distance between the convex end of each wire handle extended outside of the cannula and the distal end of the cannula is 10.00 mm, while in a frontal plane the diameter of the imaginary circumference that the 3 handles form when they are completely open or extended is 8.50 mm.

The flexibility and memory possessed by the nitinol wire added to the mechanism that allows the handles to be introduced into the 0.573 mm diameter cannula (23 gauge) or extended as a basket inside the vitreous cavity allows the vitreoretinal surgeon to work with this surgical instrument through small caliber trocars as well as to trap an intraocular foreign body or crystalline fragments of variable size and shape.

Having sufficiently described the nature of the present invention, in addition to the manner in which to put it into practice, it is hereby noted that, in its essence, it may be put into practice in other embodiments that differ in detail from that indicated by way of example, to which the protection sought likewise applies, provided that its main principle is not altered, changed or modified.

## Claims

1. Surgical instrument for vitreoretinal surgery **characterized in that** it comprises:
- a handle (1) of thermoplastic polymer,
- a stainless steel cannula (3) disposed at the distal end of the handle (1),
- a stainless steel stem (4) that runs through the interior of the cannula (3),
- three petal-shaped nitinol handles (5) attached to the distal end of the stainless steel stem (4) that runs through the interior of the cannula (3),
- a movable button (2) embedded in the handle (1) of the instrument and which slides on a rail, the stainless steel stem (4) being attached to this movable button (2) at its proximal end.
Where all the above elements allow the surgeon to extend and retract the handles (5) by means of the movable button (2) in such a way that the handles (5) are closed and inside the cannula (3) by moving the movable button (2) towards the proximal area, while by moving the movable button (2) towards the distal area it is possible to extend and open the handles (5) to the outside of the cannula gradually.

2. Surgical instrument for vitreoretinal surgery according to claim 1, **characterized in that** the cannula (3) has an external caliber of 0.573 mm in diameter (23 gauge), an internal diameter of 0.47 mm and a length of 33 mm.

3. Surgical instrument for vitreoretinal surgery according to claim 1 or 2, **characterized in that** the nitinol handles (5) are made of round monofilament nitinol wire of 0.113 mm diameter (gauge 37 gauge).

4. Surgical instrument for vitreoretinal surgery according to any one of the preceding claims, **characterized in that** the stainless steel stem has a diameter of 0.404 mm (26 gauge) and a length of 52.00 mm, at the distal end of which the nitinol handles are attached.

5. Surgical instrument for vitreoretinal surgery according to any of the preceding claims, **characterized in that** the rail above has a length of 13.00 mm.

6. Surgical instrument for vitreoretinal surgery according to any one of the preceding claims, **characterized in that** the maximum distance between the convex end of each wire handle extended outside the cannula and the distal end of the cannula 10.00 mm (23 gauge), while in a frontal plane the diameter of the imaginary circumference that the 3 handles form when they are completely open or extended is 8.50 mm.

## Amended claims

### Amended claims under Art. 19.1 PCT

1. Surgical instrument for vitreoretinal surgery **characterized in that** it comprises:
- a handle (1) of thermoplastic polymer,
- a stainless steel cannula (3) disposed at the distal end of the handle (1),
- a stainless steel stem (4) that runs through the interior of the cannula (3),
- three petal-shaped nitinol handles (5) attached to the distal end of the stainless steel stem (4) that runs through the interior of the cannula (3),
- a movable button (2) embedded in the handle (1) of the instrument and which slides on a rail, the stainless steel stem (4) being attached to this movable button (2) at its proximal end.
Where all the above elements allow the surgeon to extend and retract the handles (5) by means of the movable button (2) in such a way that the handles (5) are closed and inside the cannula (3) by moving the movable button (2) towards the proximal area, while by moving the movable button (2) towards the distal area it is possible to extend and open the handles (5) to the outside of the cannula gradually.

2. Surgical instrument for vitreoretinal surgery according to claim 1, **characterized in that** the cannula (3) has an external caliber of 0.573 mm in diameter (23 gauge), an internal diameter of 0.47 mm and a length of 33 mm.

3. Surgical instrument for vitreoretinal surgery according to claim 1 or 2, **characterized in that** the nitinol handles (5) are made of round monofilament nitinol wire of 0.113 mm diameter (gauge 37 gauge).

4. Surgical instrument for vitreoretinal surgery according to any one of the preceding claims, **characterized in that** the stainless steel stem has a diameter of 0.404 mm (26 gauge) and a length of 52.00 mm, at the distal end of which the nitinol handles are attached.

5. Surgical instrument for vitreoretinal surgery according to any of the preceding claims, **characterized in that** the rail has a length of 13.00 mm.

6. Surgical instrument for vitreoretinal surgery according to any one of the preceding claims, **characterized in that** the maximum distance between the convex end of each wire handle extended outside the cannula and the distal end of the cannula is 10.00 mm (23 gauge), while in a frontal plane the diameter of the imaginary circumference that the 3 handles form when they are completely open or extended is 8.50 mm.
